# EUROPÄISCHE PATENTSCHRIFT

(11) **EP 0 642 992 B1**
(45) Veröffentlichungstag und Bekanntmachung des Hinweises auf die Patenterteilung: **04.03.1998**
(21) Anmeldenummer: 94810478.1
(22) Anmeldetag: 18.08.1994
(51) Int. Cl.: B65D 83/14

(54) **Aerosolbehälter mit FCK-freiem Treibgas und Dosierventil sowie seine Verwendung**
Metered aerosol with CFC free propellant and dosing valve as well as application thereof
Bombe aérosol avec une valve doseuse chargée d'un gaz sans CFC et son application

(30) Priorität: 27.08.1993 EP 93810614
(43) Veröffentlichungstag der Anmeldung: 15.03.1995
(73) Patentinhaber: Novartis AG, 4058 Basel (CH)
(72) Erfinder: Brugger, François, F-68510 Waltenheim (FR); Stampf, Angelika, Dr., F-68170 Rixheim (FR)

(56) Entgegenhaltungen:
- EP-A- 0 335 315
- EP-A- 0 338 670
- WO-A-92/11190
- FR-A- 2 267 496
- US-A- 4 902 318

## Beschreibung

Die Erfindung betrifft einen Aerosolbehälter und die Verwendung eines Aerosolbehälters gemäss dem Oberbegriff des jeweiligen unabhängigen Patentanspruchs.

Aerosole sind heutzutage eine gängige Darreichungsform für pharmazeutisch wirksame Substanzen. Viele Aerosole sind dabei in vorgegebenen (dosierten) Mengen zu verabreichen. Bestimmte pharmazeutisch wirksame Substanzen werden aus verschiedenen Gründen (z.B. Stabilität) als Suspension bereitgestellt, d.h. im Aerosolbehälter liegt die pharmazeutisch wirksame Substanz in der Regel unter Druck in Form von kleinen Feststoffpartikeln in einer Flüssigkeit vor, wobei die Flüssigkeit dabei zumindest auch ein Treibgas umfasst. Diese Art der Formulierung von pharmazeutisch wirksamen Substanzen hat sich für viele Substanzen, insbesondere auch für Corticosteroide, bewährt.

Zur Darreichung einer vorgegebenen Menge der pharmazeutisch wirksamen Substanz sind gängige Aerosolbehälter mit einem Dosierventil mit einer Dosierkammer versehen. In einer ersten Stellung des Ventils steht dabei die Dosierkammer in Verbindung mit dem Innenraum des Behälters, sie ist in dieser Stellung mit der vorgegebenen Menge der Suspension befüllt. In einer zweiten Stellung des Dosierventils wird die in der Dosierkammer befindliche Menge dann in Form eines Aerosols freigegeben, da sich das Flüssigkeits-Feststoff-Gemisch entspannen kann. Auf diese Weise kann dem Benutzer das Aerosol z.B. oral oder nasal verabreicht werden.

Bisher werden die weithin bekannten Fluor-Chlor-Kohlenwasserstoffe als Treibgase eingesetzt. Diese chlorierten Treibgase sind inzwischen als schädlich bekannt, da sie die Ozonschicht abbauen. Daher sollen und müssen diese Treibgase abgeschafft bzw. durch andere für die Ozonschicht unschädliche Treibgase ersetzt werden. In einigen Ländern werden sogar in kürzester Zeit diese Treibgase, die chlorierte Kohlenwasserstoffe enthalten, gesetzlich verboten sein.

Als Alternative bieten sich daher sogenannte alternative Treibgase an, da diese nämlich die Ozonschicht nicht schädigen (Ozone-Depleting-Potential = 0). Allerdings kommt es bei der Aufbewahrung von vielen pharmazeutischen Substanzen in Form einer Suspension dann im Behälter zu Ablagerungen an der Behälterinnenwand, die bei chlorierten Kohlenwasserstoffen nicht oder nur in sehr geringem Masse aufgetreten sind. Die Folge solcher Ablagerungen an der Behälterinnenwand kann sein, dass in der Dosierkammer nicht die gewünschte Menge an pharmazeutischer Wirksubstanz vorhanden ist, die dem Benutzer verabreicht werden soll. Als weitere Folge kann die gesamte in dem Behälter aufbewahrte pharmazeutisch wirksame Substanz nicht verabreicht werden, da ein recht erheblicher Teil der gesamten in den Behälter eingefüllten pharmazeutisch wirksamen Substanz an der Behälterinnenwand angelagert bleibt (an ihr haftet).

Aus der EP-A-0,338,670 sind Aerosolbehälter aus Aluminium bekannt, die eine zu verabreichende pharmazeutisches Lösung enthalten. Um eine Kontamination der Lösung mit Metall zu verhindern, ist die Innenwand des Behälters lackiert oder anderweitig beschichtet.

FR-A-2,267,494 offenbart einen Kunststoffbehälter, der eine in den Kunststoff eingebettete dünne Metallschicht aufweist. Als Treibgase werden dort die als "Ozonkiller" bekannten Fluor-Chlor-Kohlenwasserstoffe (FCKW) angegeben.

Schliesslich ist ein weiterer gattungsgemässer Aerosolbehälter aus der WO-A-92/11190 bekannt.

Aufgabe der Erfindung ist es, einen Behälter zu schaffen, in welchem die pharmazeutisch wirksame Substanz in der bereits bewahrten Formulierung bereitgestellt wird und bei welchem es gleichzeitig möglich ist alternative, die Ozonschicht nicht schädigende, Treibgase, zu verwenden, ohne dass es dabei zu signifikanten Ablagerungen der pharmazeutisch wirksamen Substanz an der Behälterinnenwand kommt. Insbesondere soll dies möglich sein für antiasthmatisch wirksame pharmazeutische Substanzen (z.B. für Corticosteroide), allerdings sollen auch andere Klassen von pharmazeutischen Substanzen in solchen Behältern aufbewahrt werden können, ohne dass es zu signifikanten Wirksubstanzablagerungen an der Behälterinnenwand kommt.

Diese Aufgabe wird erfindungsgemäss durch einen Behälter gelöst, bei welchem die Innenwand des Behälters mit einem Kunststoffbelag aus Polytetraflourethylen, weithin als Teflon bekannt, oder mit einem Kunststoffbelag aus Perfluorethylenpropylen beschichtet ist, und bei welchem das Treibgas als alternatives Treibgas nur Fluor-Kohlenwasserstoffe und gegebenenfalls noch Cosolventien und/oder Tenside enthält. Dadurch wird erreicht, dass zum einen keine oder keine signifikanten Wirksubstanzablagerungen an der Behälterinnenwand auftreten und zum anderen die Ozonschicht nicht geschädigt bzw. abgebaut wird.

Bei speziellen Ausführungsbeispielen von solchen Behältern kann die Wandstärke der Behälterwand im Bereich von etwa 0.1 mm bis etwa 2mm und speziell etwa 0.4 mm betragen (je nach verwendetem Material), und die Dicke des Kunststoffbelags kann im Bereich von etwa 1 nm bis etwa 1 mm liegen, insbesondere kann sie einige 10 nm betragen. Die genannten Wandstärken sind für Aerosolbehälter gängig, sodass die erfindungsgemässen Aerosolbehälter sich rein äusserlich und insbesondere im Hinblick auf die äusseren Abmessungen von den bereits gängigen Behältern nicht unterscheiden und daher auch dann, wenn sie zur Darreichung des Aerosols beispielsweise in einen Applikator für gängige Aerosolbehälter eingesetzt werden müssen, problemlos verwendbar sind.

Das Volumen des Innenraums des Behälters von solchen Aerosolen liegt im Bereich von etwa 1 ml bis etwa 100 ml und das Volumen der Dosierkammer im Bereich von etwa 5 µl bis etwa 400 µl. Diese Volumina sind beispielsweise für Corticosteroide als pharmazeutisch wirksame Substanz gebräuchlich, z.B. für das Corticosteroid mit der chemischen Bezeichnung "9α-chlor-6α-fluor-11β,17α-dihydroxy-16α-methyl-3-oxo-androsta-1,4-dien-17β-carbomethoxy-17-propionat", aber auch für andere pharmazeutisch und insbesondere antiasthmatisch wirksame Substanzen wie zum Beispiel für Formoterol, welches in Form seines Salzes Formoterol fumarat vorliegen kann, dessen Bezeichnung nach der IUPAC-Nomenklatur "(±)2'-Hydroxy-5'-[(RS)-1-hydroxy-2-[[(RS)-p-methoxy-α-methylphenethyl]-amino]ethyl]formanilid · fumarat · dihydrat" lautet, oder auch für Gemische aus Formoterol und dem genannten Corticosteroid.

Besonders geeignet ist als pharmazeutisch wirksame Substanz die Substanz mit der Bezeichnung "(1R,2S)-(3E,5Z)-7-[1-(3-trifluormethylphenyl)-1-hydroxy-10-(4-acetyl-3-hydroxy-2-propylphenoxy)-3,5-decadien-2-yl-thio]-4-oxo-4H-1-benzopyran-2-carboxylsäure" bzw. ein Natriumsalz dieser Substanz, da es hier zu besonders geringen oder gar keinen Ablagerungen an der Behälterinnenwand kommt. Dabei kann das gesamte Aerosol dann 0.1%-2% dieser wirksamen Substanz sowie HFA-Treibgase (gegebenenfalls können auch Cosolventien und/oder Tenside enthalten sein) umfassen.

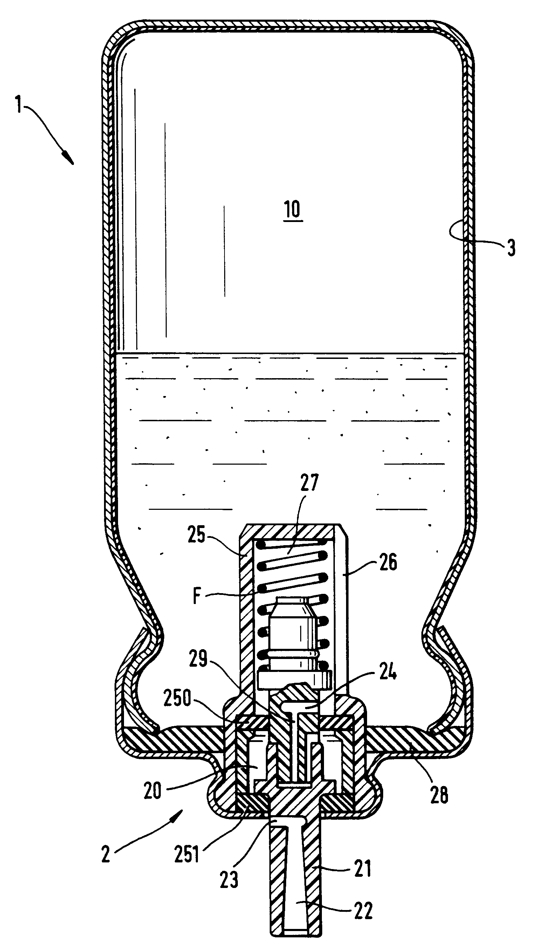

Im folgenden wird die Erfindung anhand der Zeichnung näher erläutert. Die einzige Zeichnungsfigur zeigt in schematischer Schnittdarstellung ein Ausführungsbeispiel eines erfindungsgemässen Aerosolbehälters. Der Aerosolbehälter ist dabei in seiner Gebrauchsposition dargestellt.

Der Aerosolbehälter ist generell bezeichnet mit dem Bezugszeichen 1. Die Innenwand des Behälters 1 ist mit einem Kunststoffbelag 3 beschichtet, auf dessen Bedeutung weiter unten noch detaillierter eingegangen wird. Auf dem Aerosolbehälter 1 sitzt ein Dosierventil 2 auf. Es umfasst ein Ventilröhrchen (Stem) 21, welches in einem Ventilgehäuse 25 geführt ist und gegen die Kraft einer Feder F im Ventilgehäuse 25 verschiebbar ist. In der Wand des Ventilgehäuses 25 sind einzelne Schlitze 26 vorgesehen, die den Innenraum 10 des Behälters 1 mit dem Innenraum 27 des Ventilgehäuses 25 kommunizierend verbinden. Ferner umfasst das Dosierventil 2 eine Dosierkammer 20, die durch die Schlitze 26 in der Wand des Ventilgehäuses 25 mit Hilfe des Ventilröhrchens (Stem) 21 in noch zu erläuternder Weise befüllt wird. Der Innenraum 27 des Ventilgehäuses 25 ist gegen die Dosierkammer 20 mittels einer Dichtung 250 abgedichtet; die Dosierkammer 20 wiederum ist nach aussen hin mit Hilfe einer Dichtung 251 abgedichtet. Schliesslich ist der gesamte Innenraum 10 des Behälters noch mittels einer im Dosierventil 2 vorgesehenen Dichtung 28 nach aussen hin abgedichtet.

Der Stem 21 des Dosierventils 2 weist zwei Kanäle 29 und 22 auf. Der Kanal 29 weist an seinem "inneren" Ende eine Querbohrung 24 auf, die in der dargestellten ersten Stellung des Stems 21 in den Innenraum 27 des Ventilgehäuses 25 mündet und somit den Innenraum 27 des Ventilgehäuses und damit den Innenraum 10 des Behälters mit der Dosierkammer 20 verbindet. Das Volumen der Dosierkammer 20 legt die gewünschte zu verabreichende Menge des Aerosols fest. Wie sie befüllt wird, wird später noch erläutert. In dieser ersten Stellung des Stems 21 kann jedenfalls aus der Dosierkammer 20 nach aussen hin kein Aerosol entweichen, da die Dosierkammer 20 nach aussen hin mittels der Dichtung 251 abgedichtet ist.

In der zweiten Stellung des Ventilröhrchens (Stem) 21 ist die Feder F zusammengedrückt und der Stem 21 ist so weit in den Innenraum 27 des Ventilgehäuses 25 eingetaucht, dass eine Verbindung vom Innenraum 27 des Ventilgehäuses 25 bzw. vom Innenraum 10 des Behälters 1 über den Kanal 29 nicht gegeben ist. In dieser zweiten Stellung des Stems 21 existiert aber über eine Querbohrung 23 am "inneren" Ende des Kanals 22 eine Verbindung von der Dosierkammer 20 nach aussen zum Benutzer. Die in der Dosierkammer 20 befindliche Menge an Aerosol kann sich durch diese Querbohrung 23 und den Kanal 22 hindurch entspannen und so entweder direkt oder mittels eines speziellen Applikationsgeräts dem Benutzer verabreicht werden.

Wenn nun nach der Applikation das Ventilröhrchen (Stem) 21 wieder losgelassen wird, gelangt die Querbohrung 23 nachfolgend in den Bereich der Dichtung 251, die Dosierkammer 20 ist nach aussen hin wieder abgedichtet. Der Stem 21 ist zu diesem Zeitpunkt noch nicht wieder in seiner ersten Endstellung, jedoch steht die Querbohrung 24 schon in Verbindung mit dem Innenraum 10 des Behälters 1, sodass aufgrund der Druckdifferenz (Überdruck im Behälterinnenraum, entleerte Dosierkammer) sofort Suspension aus dem Innenraum 10 des Behälters in die Dosierkammer 20 strömt und diese befüllt. Die Dosierkammer 20 wird also beim Loslassen bzw. Zurückstellen des Stems 21 sofort wieder befüllt und somit kann anschliessend sofort die nächste Applikation erfolgen.

Wie bereits einleitend erwähnt, werden viele pharmazeutisch wirksame Substanzen aus verschiedenen Gründen (z.B. Stabilität) im Behälter unter Druck als Suspension bereitsgestellt, also in Form einer Flüssigkeit, in welcher die Wirksubstanz in Form von Feststoffpartikeln enthalten ist. In der Flüssigkeit ist zumindest auch ein Treibgas enthalten, sodass sich die in der Dosierkammer 20 befindliche dosierte Menge in der zweiten Stellung des Stems 21 entspannen kann und so dem Benutzer direkt oder mittels eines speziellen Applikationsgeräts verabreicht werden kann, wie dies bereits oben erläutert ist.

Da das Treibgas Fluorkohlenwasserstoffe (vorzugsweise z.B. Tetrafluorethan oder Heptafluorpropan) umfasst und somit unschädlich für die Ozonschicht ist, ist die Innenwand des Behälters 1 mit einem Kunststoffbelag 3 beschichtet. Dieser Kunststoffbelag 3 ist vorzugsweise aus Polytetrafluorethylen, weithin auch unter dem Namen Teflon bekannt, oder aus Perfluorethylenpropylen oder die Schicht ist auf einer Basis des jeweiligen Kunststoffs hergestellt und aufgebracht. Bei der Verwendung dieser Materialien werden signifikante Ablagerungen der Wirksubstanz an der Innenwand des Behälters 1 vermieden. Ebenso werden Korrosions- und Elektrolyseeffekte zwischen Behälterwand und Flüssigkeit bzw. Suspension vermieden.

Zur Beschichtung der Innenwand des Behälters 1 mit dem Kunststoffbelag 3 können die unterschiedlichsten Verfahren zum Einsatz kommen. Beispielsweise können als Beschichtungsverfahren eine Plasmabeschichtung, Imprägnier-/Spritzverfahren, Hartanodisieren mit PTFE-Einlagerung, Chemical Vapour Deposition (CVD) , Physical Vapour Deposition (PVD) und andere zu diesem Zweck gängige Verfahren zum Einsatz kommen. Besonders bevorzugt wird die Plasmabeschichtung eingesetzt.

Die Behälterwand kann beispielsweise aus Aluminium hergestellt sein. Ihre Wandstärke liegt beispielsweise im Bereich von etwa 0.1 mm bis etwa 2mm und beträgt vorzugsweise etwa 0.4 mm. Die Dicke des Kunststoffbelags liegt im Bereich von etwa 1 nm bis etwa 1 mm und beträgt vorzugsweise etwa einige 10 nm bis einige 10 µm. Diese Wandstärken sind typisch für Behälter, deren gesamtes Behältervolumen im Bereich von 1 ml bis etwa 100 ml liegt und vorzugsweise etwa 5 ml bis etwa 20 ml beträgt. Das Dosiervolumen, also das Volumen der Dosierkammer 20, liegt dabei beispielsweise im Bereich von etwa 5 µl bis etwa 400 µl und beträgt vorzugsweise etwa 25 µl bis etwa 200 µl.

Die zu verabreichende Wirksubstanz kann beispielsweise ein antiasthmatisch wirksamer Stoff bzw. Stoffgemisch sein, insbesondere ein Stoff bzw. Stoffgemisch aus der Klasse der Corticosteroide bzw. der entzündungshemmenden Steroide. Speziell kann es sich bei dem Corticosteroid um das Corticosteroid mit der chemischen Bezeichnung "9α-chlor-6α-fluor-11β,17α-dihydroxy-16α-methyl-3-oxo-androsta-1,4-dien-17β-carbomethoxy-17-propionat" handeln. Ebensogut ist aber auch die Verabreichung von anderen pharmazeutisch und insbesondere antiasthmatisch wirksamen Substanzen denkbar wie β-Sympathomimetica, LTD₄-Antagonisten, Parasympatholytica, Cromoglycinsäure oder von anderen Wirksubstanzen, die via Lunge, Nase oder Rachen verabreicht werden, wie dies bei einigen Proteinen der Fall ist. Des weiteren ist es beispielsweise auch denkbar, Formoterol z.B. in Form seines Salzes Formoterol fumarat, dessen Bezeichnung nach der IUPAC-Nomenklatur "(±)2'-Hydroxy-5'-[(RS)-1-hydroxy-2-[[(RS)-p-methoxy-α-methylphenethyl]-amino]ethyl]formanilid · fumarat · dihydrat" lautet, auf diese Weise aufzubewahren und zu verabreichen oder ein Gemisch aus Formoterol und dem genannten Corticosteroid.

Ganz besonders geeignet als pharmazeutisch wirksame Substanz ist auch die Substanz mit der Bezeichnung "(1R,2S)-(3E,5Z)-7-[1-(3-trifluormethylphenyl)-1-hydroxy-10-(4-acetyl-3-hydroxy-2-propylphenoxy)-3,5-decadien-2-yl-thio]-4-oxo-4H-1-benzopyran-2-carboxylsäure" bzw. ein Natriumsalz dieser Substanz, da es hier zu besonders geringen oder gar keinen Ablagerungen an der Behälterinnenwand kommt. Dabei kann das gesamte Aerosol dann 0.1%-2% dieser wirksamen Substanz sowie HFA-Treibgase (gegebenenfalls können auch Cosolventien und/oder Tenside enthalten sein) umfassen.

## Patentansprüche

1. Aerosolbehälter, welcher mit pharmazeutisch wirksamen, in vorgegebenen Mengen zu verabreichenden Aerosolen, gefüllt ist, die in Form einer Suspension in dem Behälter bereitgestellt werden, wobei die Suspension ausser einer pharmazeutisch wirksamen Substanz zumindest noch ein Treibgas umfasst, mit einem Dosierventil, welches eine Dosierkammer und ein Ventilröhrchen aufweist, wobei die Dosierkammer in einer ersten Stellung des Ventilröhrchens mit dem Innenraum des Behälters in Verbindung steht und mit einer vorgegebenen Menge des Aerosols befüllt ist und in einer zweiten Stellung des Ventilröhrchens die in der Dosierkammer befindliche Menge des Aerosols freigibt, dadurch gekennzeichnet, dass das Treibgas nur
Fluor-Kohlenwasserstoffe und gegebenenfalls noch Cosolventien und/oder Tenside umfasst, und dass die Innenwand des Behälters mit einem Kunststoffbelag aus Polytetrafluorethylen oder Perfluorethylenpropylen beschichtet ist.

2. Aerosolbehälter nach Anspruch 1, dadurch gekennzeichnet, dass die Wandstärke der Behälterwand im Bereich von etwa 0.1 mm bis etwa 2 mm liegt und insbesondere etwa 0.4 mm beträgt und die Dicke des Kunststoffbelags im Bereich von etwa 1 nm bis etwa 1 mm liegt und insbesondere einige 10 nm beträgt.

3. Aerosolbehälter nach einem der Ansprüche 1 oder 2, dadurch gekennzeichnet, dass das Volumen des Innenraums des Behälters im Bereich von etwa 1 ml bis etwa 100 ml liegt und das Volumen der Dosierkammer etwa 5 µl bis etwa 400 µl beträgt.

4. Verwendung eines Aerosolbehälters nach einem der vorangehenden Ansprüche zum Aufbewahren und Verabreichen einer vorgegebenen Menge von pharmazeutisch wirksamen Aerosolen in Form einer Suspension.

5. Verwendung nach Anspruch 4, dadurch gekennzeichnet, dass die in der Suspension enthaltene pharmazeutisch wirksame Substanz ein antiasthmatisch wirkender Stoff oder Stoffgemisch ist.

6. Verwendung nach Anspruch 5, dadurch gekennzeichnet, dass die in der Suspension enthaltene pharmazeutisch wirksame Substanz Formoterol oder ein Corticosteroid ist, insbesondere 9α-chlor-6α-fluor-11β,17α-dihydroxy-16α-methyl-3-oxo-androsta-1,4-dien-17β-carbomethoxy-17-propionat, oder ein Gemisch aus Formoterol und diesem Corticosteroid.

7. Verwendung nach Anspruch 5, dadurch gekennzeichnet dass die pharmazeutisch wirksame Substanz (1R,2S)-(3E,5Z)-7-[1-(3-trifluormethylphenyl)-1-hydroxy-10-(4-acetyl-3-hydroxy-2-propylphenoxy)-3,5-decadien-2-yl-thio]-4-oxo-4H-1-benzopyran-2-carboxylsäure ist.

## Claims

1. An aerosol container filled with pharmaceutically active aerosols that are to be administered in predetermined amounts and that are supplied in the container in the form of a suspension, the suspension also comprising, in addition to a pharmaceutically active substance, at least a propellant gas, which aerosol container has a metering valve that comprises a metering chamber and a valve stem, the metering chamber being in communication with the interior of the container and being full of a predetermined amount of the aerosol in a first position of the valve stem, and releasing the amount of aerosol disposed in the metering chamber in a second position of the valve stem, wherein the propellant gas comprises only fluorohydrocarbons and, where appropriate, also cosolvents and/or surfactants, and wherein the inner wall of the container is coated with a plastics coating of polytetrafluoroethylene or perfluoroethylenepropylene.

2. An aerosol container according to claim 1, wherein the thickness of the container wall is in the range from approximately 0.1 mm to approximately 2 mm, and is especially approximately 0.4 mm, and the thickness of the plastics coating is in the range from approximately 1 nm to approximately 1 mm, and is especially some 10 nm.

3. An aerosol container according to either claim 1 or claim 2, wherein the volume of the interior of the container is in the range from approximately 1 ml to approximately 100 ml and the volume of the metering chamber is from approximately 5 µl to approximately 400 µl.

4. The use of an aerosol container according to any one of the preceding claims for the storage and administration of a predetermined amount of a pharmaceutically active aerosol in the form of a suspension.

5. The use according to claim 4, wherein the pharmaceutically active substance in the suspension is an anti-asthmatically active substance or substance mixture.

6. The use according to claim 5, wherein the pharmaceutically active substance in the suspension is Formoterol or a corticosteroid, especially 9α-chloro-6α-fluoro-11β,17α-dihydroxy-16α-methyl-3-oxo-androsta-1,4-diene-17β-methoxycarbonyl-17-propionate, or a mixture of Formoterol and that corticosteroid.

7. The use according to claim 5, wherein the pharmaceutically active substance is (1R,2S)-(3E,5Z)-7-[1-(3-trifluoromethylphenyl)-1-hydroxy-10-(4-acetyl-3-hydroxy-2-propylphenoxy)-3,5-decadien-2-ylthio]-4-oxo-4H-1-benzopyrane-2-carboxylic acid.

## Revendications

1. Une bombe aérosol qui contient des aérosols pharmaceutiquement actifs destinés à être administrés en quantités pré-déterminées, les aérosols étant présents dans la bombe sous la forme d'une suspension qui comprend, outre une substance pharmaceutiquement active, au moins un gaz pulseur, la bombe aérosol ayant une valve doseuse comportant une chambre de dosage et une tige de la valve, la chambre de dosage communiquant avec l'intérieur de la bombe aérosol dans une première position de la tige de la valve et étant remplie d'une quantité pré-déterminée d' aérosol et libérant la quantité d' aérosol déposée dans la chambre de dosage dans une seconde position de la tige de la valve, caractérisée en ce que le gaz pulseur comprend uniquement des hydrocarbures fluorés et éventuellement des co-solvants et/ou des tensio-actifs et en ce que la paroi interne de la bombe aérosol est revêtue d'un revêtement plastique en polytétrafluoroéthylène ou en perfluoroéthylènepropylène.

2. Une bombe aérosol selon la revendication 1, caractérisée en ce que l'épaisseur de la paroi de la bombe aérosol est de l'ordre d'environ 0,1 mm à environ 2 mm et se situe en particulier à environ 0,4 mm et en ce que l'épaisseur du revêtement plastique est de l'ordre d'environ 1 nm à environ 1 mm et se situe en particulier à quelque 10 nm.

3. Une bombe aérosol selon la revendication 1 ou 2, caractérisée en ce que le volume de l'intérieur de la bombe aérosol est de l'ordre d'environ 1 ml à environ 100 ml et en ce que le volume de la chambre de dosage est de l'ordre d'environ 5 µl à environ 400 µl.

4. L'utilisation d'une bombe aérosol selon l'une quelconque des revendications précédentes pour le stockage et l'administration d'une quantité prédéterminée d'aérosols pharmaceutiquement actifs sous la forme d'une suspension.

5. L'utilisation selon la revendication 4, caractérisée en ce que la substance pharmaceutiquement active contenue dans la suspension est une substance ou un mélange de substances efficace du point de vue antiasthmatique.

6. L'utilisation selon la revendication 5, caractérisée en ce que la substance pharmaceutiquement active contenue dans la suspension est le Formoterol ou un corticostéroïde, spécialement le 9α-chloro-6α-fluoro-11β,17α-dihydroxy-16α-méthyl-3-oxo-androsta-1,4-diène-17β-carbométhoxy-17-propionate ou un mélange de Formoterol et de ce corticostéroïde.

7. L'utilisation selon la revendication 5, caractérisée en ce que la substance pharmaceutiquement active est l'acide (1R,2S)-(3E,5Z)-7-[1-(3-trifluorométhylphényl)-1-hydroxy-10-(4-acétyl-3-hydroxy-2-propylphénoxy)-3,5-décadiène-2-yl-thio]-4-oxo-4H-1-benzopyranne-2-carboxylique.
